# EUROPÄISCHE PATENTSCHRIFT

(11) **EP 2 313 010 B1**
(45) Veröffentlichungstag und Bekanntmachung des Hinweises auf die Patenterteilung: **05.06.2019**
(21) Anmeldenummer: 08782820.8
(22) Anmeldetag: 19.08.2008
(51) Int. Cl.: A61B 17/04, A61B 17/12, A61B 17/06

(54) **CHIRURGISCHE EINRICHTUNG**
SURGICAL DEVICE
DISPOSITIF CHIRURGICAL

(43) Veröffentlichungstag der Anmeldung: 27.04.2011
(73) Patentinhaber: A.M.I. Agency for Medical Innovations GmbH, 6800 Feldkirch (AT)
(72) Erfinder: EGLE, Walter, A-6842 Koblach (AT); ERHARD, Martin, A-6780 Silbertal (AT)
(74) Vertreter: Hofmann, Ralf U.
(86) Internationale Anmeldenummer: PCT/AT2008/000292
(87) Internationale Veröffentlichungsnummer: WO 2010/019973

(56) Entgegenhaltungen:
- EP-A- 1 297 787
- WO-A-02/07609
- US-A- 3 470 875
- US-A- 6 077 276
- US-A1- 2003 208 209
- US-A1- 2003 216 613
- US-A1- 2008 114 382
- US-A1- 2008 147 095

## Beschreibung

Die Erfindung bezieht sich auf eine chirurgische Einrichtung umfassend ein Instrument, das ein Schaftteil und ein an einem distalen Ende des Schaftteils angeordnetes Maulteil mit einer Maulöffnung aufweist, einen Fadenmitnehmer, der vom Schaftteil geführt ist und von einer zurückgezogenen Position, in der er die Maulöffnung freigibt, in eine vorgeschobene Position, in der er die Maulöffnung überquert und mit einem distalen Ende durch eine distal der Maulöffnung gelegene Durchtrittsöffnung des Maulteils ragt, verschiebbar ist, und einen Faden, der mittels des Fadenmitnehmers durch ein menschliches oder tierisches Gewebe, in dem eine Naht anzubringen ist, oder um ein menschliches oder tierisches Gewebe herum, um das eine Ligatur anzulegen ist, führbar ist, wobei der am Faden endseitig mit einer Verdickung versehen ist, die vom Fadenmitnehmer beim Verschieben des Fadenmitnehmers von seiner zurückgezogenen in seine vorgeschobene Position mitgenommen wird.

Chirurgische Einrichtungen zum Nähen sind in unterschiedlichen Ausführungsformen bekannt geworden. Meist ist ein Instrument vorhanden, mittels dem eine Nadel, an der der Faden angebracht ist, durch das zu nähende Gewebe vorgetrieben wird, worauf die Nadel mitsamt dem Faden durch den geschaffenen Gewebekanal durchgezogen werden kann, sodass der Faden für das anschließende Verknoten vorgelegt ist. Hierbei wird das vordere Ende der durch das Gewebe durchgestoßenen Nadel mit einem geeigneten Greifinstrument gefasst. Eine derartige Näheinrichtung geht beispielsweise aus der WO 92/12674 A1 hervor.

Aus der EP 1 306 056 B1 ist eine chirurgische Einrichtung bekannt, bei der eine Nadel zwischen einer zurückgezogenen Stellung und einer vorgeschobenen Stellung verschoben werden kann, in welcher sie eine Maulöffnung in einem Maulteil des Instruments überquert und durch eine distal der Maulöffnung gelegene Durchtrittsöffnung im Maulteil ragt. Die Nadel besitzt eine Art Widerhaken, durch welchen sie beim anschließenden Zurückziehen eine die Maulöffnung im Maulteil überquerende Schlaufe des Fadens mitnimmt, sodass die Nadel einen Fadenmitnehmer darstellt. Eines der beiden Fadenenden kann in der Folge durch das Gewebe durchgezogen werden, worauf der Faden für das anschließende Verknoten vorgelegt ist. Nachteilig an dieser Einrichtung ist die eher komplizierte Handhabung. Auch ist für das Zurückziehen der Nadel mit der eingehängten Fadenschlaufe eine entsprechende Größe des im Gewebe geschaffenen Kanals erforderlich, der zu einer entsprechenden Traumatisierung führt.

Eine chirurgische Einrichtung der eingangs genannten Art ist aus einem der in der WO 02/07609 A2 beschriebenen Ausführungsbeispiele bekannt. Am Ende des Fadens ist ein Armierungsteil festgelegt, welches in Form einer Nadelspitze ausgebildet ist. Ein distal der Maulöffnung liegender Abschnitt des Maulteils ist endseitig mit einem Schnappmechanismus für das Armierungsteil ausgebildet, der von gabelartigen Fortsätzen am Ende des Maulteils gebildet wird. Nach dem Durchdrücken des Armierungsteils durch die Durchtrittsöffnung wird das Armierungsteil von diesem Schnappmechanismus gegen ein Zurückziehen gesichert.

Aufgabe der Erfindung ist es eine einfach handhabbare Einrichtung der eingangs genannten Art bereitzustellen. Erfindungsgemäß gelingt dies durch eine chirurgische Einrichtung mit den Merkmalen des Anspruchs 1. Besondere Ausführungsformen der Erfindung sind in den Unteransprüchen angegeben.

Bei der Einrichtung der Erfindung ist am Faden endseitig aus dem Material des Fadens eine Verdickung ausgebildet, die vom Fadenmitnehmer mitgenommen wird, wenn dieser von seiner zurückgezogenen Position in seine vorgeschobene Position verschoben wird. Hierbei wird die Verdickung durch die Durchtrittsöffnung im Maulteil gedrückt, wobei sich die Verdickung teilweise oder vollständig elastisch verformt oder eine Kombination der genannten Verformung der Verdickung und einer teilweisen oder vollständigen elastischen Verformung eines die Durchtrittsöffnung im Maulteil teilweise oder vollumfänglich begrenzenden Materials auftritt. Wenn der Fadenmitnehmer im Weiteren wieder zurückgezogen wird, so wird die am Faden angeordnete Verdickung vom Rand der Durchtrittsöffnung im Maulteil zurückgehalten, also nicht mit dem Fadenmitnehmer zurückgeführt. Wenn im Weiteren das Instrument zurückgezogen wird, so wird dadurch der Faden für ein Verknüpfen der Naht vorgelegt bzw. zum Setzen der Ligatur um das Gewebe herumgeführt.

Mit der Einrichtung der Erfindung kann somit sehr einfach ein Faden für eine Naht vorgelegt werden oder zum Setzen einer Ligatur um Gewebe herumgeführt werden. Wenn der Faden durch Gewebe durchzuführen ist, so erfolgt eine geringe Traumatisierung des Gewebes, da der im Gewebe geschaffene Stichkanal sehr klein sein kann.

Der Fadenmitnehmer ist vorteilhafterweise röhrchenförmig ausgebildet, wobei der Faden durch den inneren Kanal des Röhrchens verläuft und die am Faden angeordnete Verdickung vor dem distalen Ende des Röhrchens liegt und hierbei der Außendurchmesser der Verdickung größer ist als der Innendurchmesser des Kanals, sodass die Verdickung nicht durch den Kanal zurückgezogen werden kann. Beim Vorschieben des Fadenmitnehmers wird somit das die Verdickung aufweisende Fadenende vom Fadenmitnehmer mitgenommen. Beim Zurückziehen des Fadenmitnehmers kann der Faden aus dem distalen Ende des Kanals teilweise oder vollständig herausgezogen werden.

Der Fadenmitnehmer ist vorzugsweise in einem inneren Kanal des Schaftteils des Instruments geführt, wobei er in der zurückgezogenen Position mitsamt der am Faden angeordneten Verdickung im inneren Kanal des Schaftteils angeordnet ist. Wenn der Fadenmitnehmer in seine vorgeschobene Position verschoben wird, so ragt er mit einem Endabschnitt, der die Maulöffnung des Maulteils überquert, aus dem inneren Kanal heraus. Nach dem "Einhängen" der am Faden angeordneten Verdickung in der Durchtrittsöffnung des Maulteils wird der Fadenmitnehmer wieder soweit zurückgezogen, dass er innerhalb des Kanals im Schaftteil liegt oder bis er proximal aus diesem herausgezogen ist.

Die am Ende des Fadens angeordnete, vom Fadenmitnehmer beim Vorschieben mitgenommene Verdickung wird vom Material des Fadens selbst gebildet. Bei einem aus schmelzfähigem Kunststoff ausgebildeten Faden kann die Verdickung durch Aufschmelzen des Fadenendes, sodass ein verdickter Kopf entsteht, gebildet werden. Es existieren gängige chirurgische Fäden, die aus einem solchen schmelzbaren Kunststoff bestehen. Eine weitere Variante besteht darin, das Fadenende, z.B. durch einfaches Umknicken, so zu verformen und in dieser Verformung zu halten (beispielsweise durch eine Verklebung), dass sich eine Verdickung bildet.

Eine Einrichtung der Erfindung kann zur Anbringung einer chirurgischen Naht, speziell Einzelknopfnaht, dienen, wobei der Faden mit der erfindungsgemäßen chirurgischen Einrichtung vorgelegt wird, d.h. durch das zu nähende Gewebe durchgeführt wird. Weiters oder alternativ kann eine solche chirurgische Einrichtung zum Setzen einer Ligatur im menschlichen oder tierischen Gewebe dienen, d.h. zum Abbinden von Gewebepürzeln, Gefäßen oder anderen Hohlorganen, wobei der Faden um das abzubindende Gewebe herumgeführt wird.

Wenn in dieser Schrift von "proximal" und "distal" die Rede ist, so ist damit die Lage zum Operateur bezeichnet, ein distales Teil liegt weiter vom Operateur entfernt als ein demgegenüber proximales Teil. Weiters wird eine Bewegung von proximal nach distal als nach vorne und eine umgekehrte Bewegung als nach hinten bzw. zurückgerichtet bezeichnet.

Weitere Vorteile und Einzelheiten der Erfindung werden im Folgenden anhand der beiliegenden Zeichnung erläutert. In dieser zeigen:
Fig. 1 eine Schrägsicht eines Instruments und eines in dieses einzuführenden Fadenmitnehmers, in den ein Faden eingesetzt ist, gemäß einer möglichen Ausbildungsform der Erfindung;
Fig. 2 Eine Schrägsicht der in Fig. 1 dargestellten Einrichtung aus einem geänderten Blickwinkel, wobei der Mitnehmer in das Instrument eingesetzt ist und sich in seiner vorgeschobenen Position befindet;
Fig. 3 ein vergrößertes Detail A von Fig. 1;
Fig. 4 einen Längsmittelschnitt durch das Instrument, ohne den eingesetzten Mitnehmer;
Fig. 5 einen Längsschnitt entsprechend Fig. 4, mit eingesetztem und mit einem fadengeladenen Mitnehmer, der sich in seiner zurückgezogenen Position befindet;
Fig. 6 einen Längsschnitt analog Fig. 5, aber in der vorgeschobenen Position des Mitnehmers;
Fig. 7 ein vergrößertes Detail B von Fig. 6;
Fig. 8 bis 12 Darstellungen zur Verdeutlichung der einzelnen Schritte beim Vorlegen eines Fadens, wobei die Einrichtung jeweils im Längsmittelschnitt dargestellt ist;
Fig. 13 eine schematische Darstellung eines distalen Endabschnitts des Instruments gemäß einer weiteren Ausführungsvariante der Erfindung.

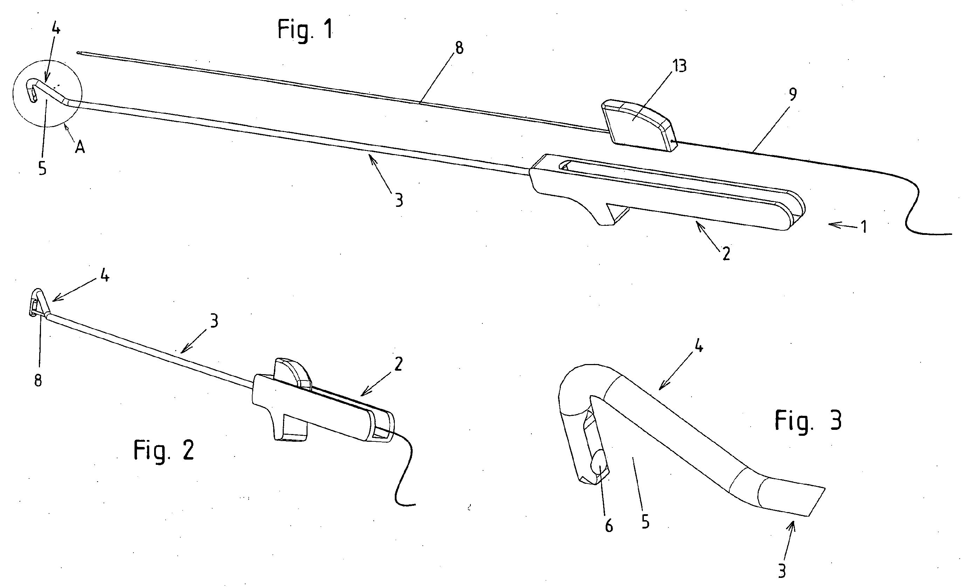

Ein erstes Ausführungsbeispiel der Erfindung wird im Folgenden anhand der Fig. 1 bis 12 erläutert. Die Einrichtung umfasst ein Instrument 1 mit einem Griffteil 2, an dem das Instrument gehalten werden kann, einem längserstreckten, vorzugsweise geraden, Schaftteil 3 und einem am distalen Ende des Schaftteils 3 angeordnetes Maulteil 4, das eine Maulöffnung 5 aufweist. Die Maulöffnung 5 öffnet sich bezogen auf die Längsachse 16 des Schaftteils 3 zur Seite hin (also nicht nach vorne).

Durch das Schaftteil 3 und durch das Maulteil 4 bis zur Maulöffnung 5 verläuft ein innerer Kanal 7. Der Kanal 7 mündet also distal in die Maulöffnung 5 und ist an seinem proximalen Ende, welches am proximalen Ende des Schaftteils 3 oder im Bereich des Griffteils 2 liegen kann, ebenfalls offen. Entsprechend der geraden Ausbildung des Schaftteils 3 verläuft der Kanal 7 geradlinig und in der geradlinigen Verlängerung des Kanals 7 liegt distal der Maulöffnung 5 eine einen distalen Abschnitt des Maulteils 4 ausgehend vom Bereich der Maulöffnung 5 geradlinig und parallel zur Richtung der Längserstreckung des Schaftteils 3 durchsetzende Durchtrittsöffnung 6.

Das Maulteil 4 und das Schaftteil 3 werden im gezeigten Ausführungsbeispiel von einem gemeinsamen Schaft des Instruments gebildet, dessen Endabschnitt zur Ausbildung des Maulteils 4 einen gebogenen Verlauf aufweist. Hierbei umfasst der Verlauf erste und zweite Biegungen in entgegengesetzte Richtungen, um die Maulöffnung 5 auszubilden. Die Durchtrittsöffnung 6 ist im der Mündung des Kanals 7 gegenüberliegenden Endabschnitt des Schaftes angeordnet, der winkelig, beispielsweise annähernd rechtwinkelig, zum Kanal 7 verläuft.

In den Kanal 7 ist ein röhrchenförmiger Fadenmitnehmer 8 einführbar, und zwar vom proximalen Ende des Kanals 7 her. Der in den Fig. 1, 2 und 5-7 dargestellte Fadenmitnehmer 8 ist mit einem Faden 9 beladen. Hierbei erstreckt sich der Faden 9 durch einen inneren Fadenkanal 10 des Fadenmitnehmers 8. Am vorderen Ende des Fadens 9 weist dieser eine Verdickung 11 auf, die vor dem distalen Ende des Fadenkanals 10 liegt und hierbei so groß ist, dass sie nicht in den Fadenkanal 10 hineingezogen werden kann. Die Verdickung 11 kann beispielsweise aus dem Material des Fadens 9 bestehen, wobei sie durch ein Aufschmelzen des Kunststoffmaterials des Fadens 9 gebildet ist. Das hintere Ende des Fadens 9 kann wie dargestellt aus dem proximalen Ende des Fadenkanals 10 herausragen oder auch innerhalb des Fadenkanals 10 liegen.

Das distale Ende des Fadenmitnehmers 8 kann verjüngt ausgebildet sein, sodass der Fadenmitnehmer 8 hohlnadelförmig ausgebildet ist, wodurch ein Durchstechen von Gewebe erleichtert wird.

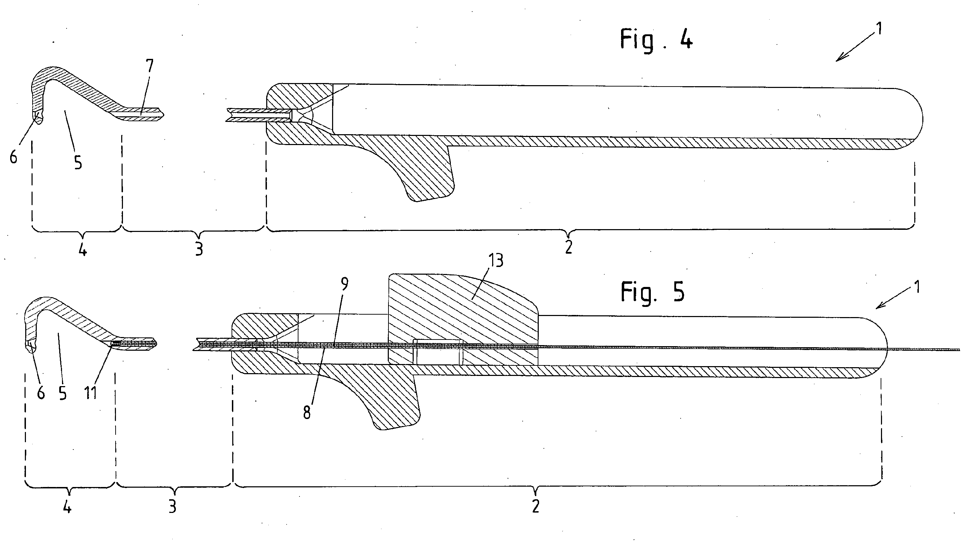

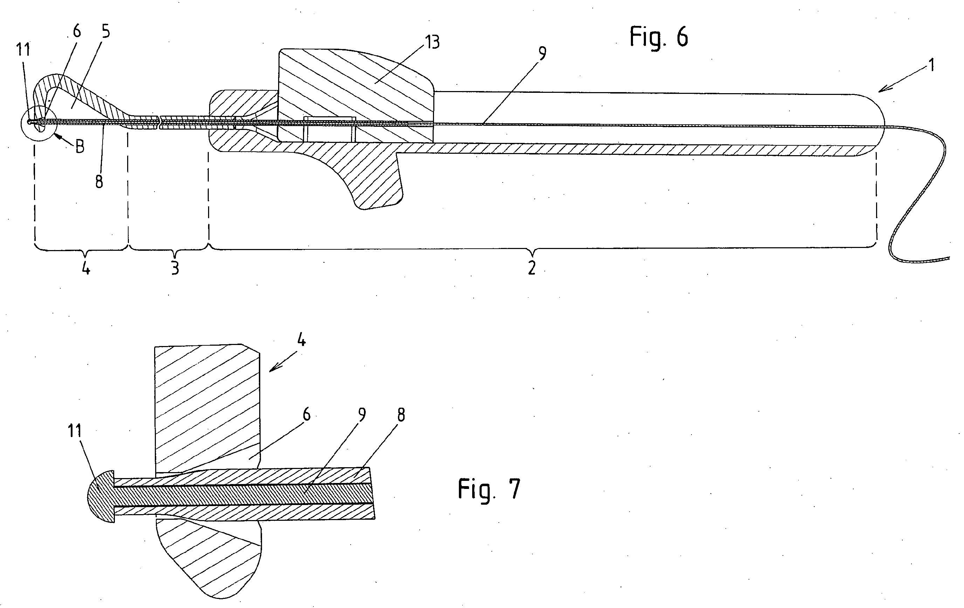

Der Fadenmitnehmer 8 kann von einer zurückgezogenen Position (Fig. 5) in eine vorgeschobenen Position (Fig. 6) in Richtung der Längsachse 16 des Schaftteils 3 verschoben werden. In der zurückgezogenen Position befindet sich das distale Ende des Fadenmitnehmers 8 innerhalb des Kanals 7 des Instruments 1. Im gezeigten Ausführungsbeispiel kann das distale Ende des Fadenmitnehmers 8 beliebig weit in den Kanal 7 zurückgezogen werden bzw., um ein vollständiges Trennen des Fadenmitnehmers 8 vom Instrument 1 zu ermöglichen, aus dem proximalen Ende des Kanals 7 herausgezogen werden.

In der vorgeschobenen Position des Fadenmitnehmers 8 ragt er mit einem Endabschnitt aus dem Kanal 7 heraus, wobei dieser Endabschnitt die Maulöffnung 5 überquert und mit seinem proximalen Ende durch die Durchtrittsöffnung 6 ragt.

Beim Verschieben des Mitnehmers 8 von seiner zurückgezogenen Position in seine vorgeschobene Position nimmt der Fadenmitnehmer 8 die Verdickung 11 mit und führt diese durch die Durchtrittsöffnung 6 hindurch. Hierbei wird die Verdickung zusammengepresst. Die Verformung der Verdickung 11 ist hierbei zumindest teilweise elastisch. Nach ihrem Durchtritt durch die Durchtrittsöffnung 6 weitet sich die Verdickung 11 daher wieder auf, sodass sie einen größeren Durchmesser als die Durchgangsöffnung 6 im distalen Mündungsbereich aufweist und sie bei einem anschließenden Zurückziehen des Fadenmitnehmers 8 gegen ein Zurückziehen durch die Durchtrittsöffnung 6 gehalten ist. Beim Zurückziehen des Fadenmitnehmers 8 wird das distale Ende des Fadens 9 somit aus dem distalen Ende des Fadenmitnehmers 8 herausgezogen.

Die Durchtrittsöffnung 6 im distal der Maulöffnung 5 liegenden Abschnitt des Maulteils 4 erweitert sich zu ihrer bei der Maulöffnung 5 liegenden Mündung hin, um eine größere Toleranz für das Einführen des Fadenmitnehmers 8 zu erhalten. Insbesondere wird eine Art Einlaufkonus für den Fadenmitnehmer 8 ausgebildet.

Zum Verschieben des Fadenmitnehmers 8 dient im gezeigten Ausführungsbeispiel ein an diesem angebrachtes Betätigungsteil 13.

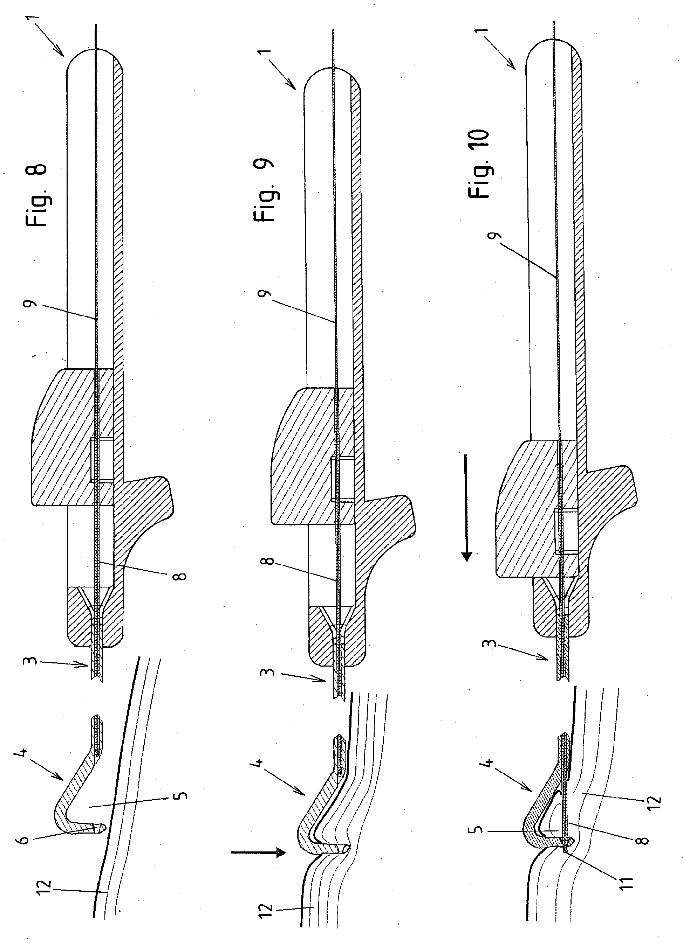

In den Fig. 8 bis 12 ist die Anwendung der Einrichtung zum Vorlegen eines Fadens zur Ausbildung einer chirurgischen Einzelknopfnaht dargestellt. Nachdem das freie Ende des distal der Maulöffnung 5 liegenden, die Durchtrittsöffnung 6 aufweisenden Abschnitt des Maulteils 4, welches als Einstechspitze ausgebildet ist, an das menschliche oder tierische Gewebe 12, in dem die Naht herzustellen ist, herangeführt worden ist (Fig. 8), wird der distal der Maulöffnung 5 liegende Abschnitt des Maulteils 4 in das Gewebe 12 eingestochen, wie dies aus Fig. 9 ersichtlich ist. Dadurch gelangt Gewebe 12 in den Bereich der Maulöffnung 5. Der Fadenmitnehmer befindet sich hierbei in einer zurückgezogenen Position, in der sein distales Ende innerhalb des Kanals 7 liegt.

In der Folge wird der Fadenmitnehmer 8 durch Vordrücken des Betätigungsteils 13 nach distal (=nach vorne) verschoben, wobei er den Faden 9 mitnimmt. Das distale Ende des Fadenmitnehmers 8 und die vor diesem liegende Verdickung 11 des Fadens 9 werden hierbei durch das Gewebe 12 gestochen, wobei ein Durchtrittskanal für den Faden 9 geschaffen wird. Der Fadenmitnehmer 8 wird bis in seine in Fig. 10 dargestellte vorgeschobene Position verschoben, in welcher er durch die Durchtrittsöffnung 6 im distalen Abschnitts des Maulteils 4 tritt, wie dies in Fig. 10 dargestellt ist. Die Verdickung 11 des Fadens 9 ist dadurch durch die Durchtrittsöffnung 6 gedrückt worden.

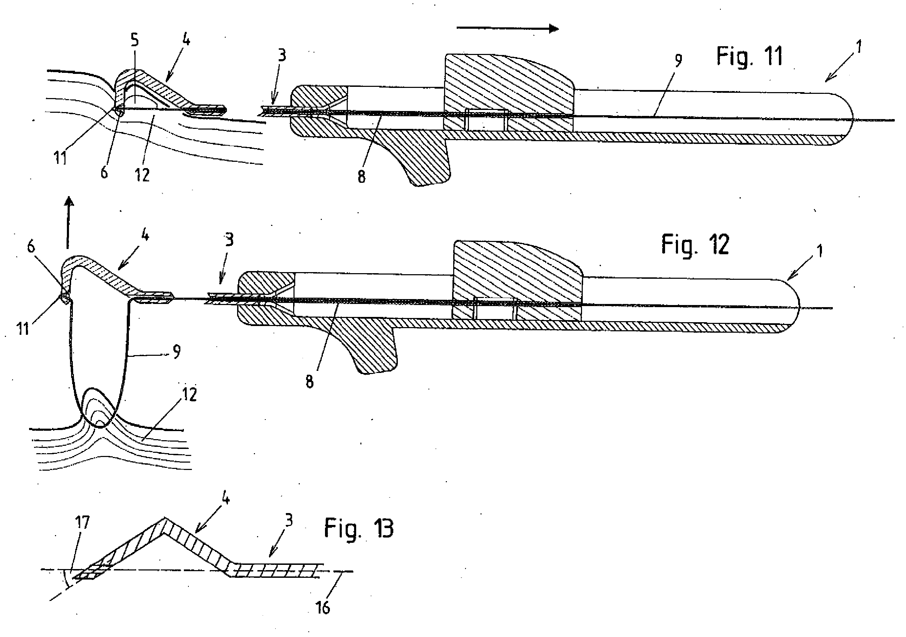

In der Folge wird der Fadenmitnehmer 8 wiederum in eine zurückgezogene Position verschoben, in der sein distales Ende im Kanal 7 liegt, wie dies in Fig. 11 dargestellt ist. Es kann sich hierbei um die ursprüngliche zurückgezogene Position oder um eine andere zurückgezogene Position handeln (d.h. das distale Ende des Fadenmitnehmers 8 befindet sich an einer anderen Position wie vor dem Vorschieben). Die Verdickung 11 im Faden wird hierbei vom distalen Abschnitt des Maulteils 4 zurückgehalten, bewegt sich also nicht durch die Durchtrittsöffnung 6 zurück. Beim Zurückschieben des Fadenmitnehmers 8 bleibt der Faden 9 somit stationär, wobei er durch den Fadenkanal 10 des Fadenmitnehmers 8 gezogen wird. Die Situation nach dem Zurückziehen des Fadenmitnehmers 8 ist in Fig. 11 dargestellt.

Das Zurückziehen des Fadenmitnehmers 8 erfolgt im gezeigten Ausführungsbeispiel durch das Betätigungsteil 13. Denkbar und möglich ist es beispielsweise auch, den Fadenmitnehmer 8 durch mindestens ein Federelement zu beaufschlagen, derart, dass der Fadenmitnehmer 8 von diesem mindestens einen Federelement von seiner vorgeschobenen Position in eine zurückgezogene Position verschoben werden kann, in welcher sein distales Ende innerhalb des Kanals 7 liegt.

In der Folge wird das Maulteil 4 aus dem Gewebe 12 herausgezogen. Hierbei wird die Verdickung 11 nach wie vor vom distalen Abschnitt des Maulteils 4 gehalten. Der Faden 9 wird somit durch den Gewebekanal gezogen, wobei er weiter durch den Fadenkanal 10 des Fadenmitnehmers 8 und aus dem Kanal 7 des Instruments 1 herausgezogen wird. Diese Situation ist in Fig. 12 dargestellt. Das Herausziehen des Instruments 1 wird solange fortgesetzt, bis die beiden aus dem Gewebekanal herausragenden Fadenenden lang genug sind. In der Folge wird zumindest das an die Verdickung 11 anschließende Fadenende abgeschnitten und, falls das andere Fadenende noch nicht vollständig aus dem Instrument 1 herausgezogen worden ist, auch dieses. Der Faden ist nunmehr vorgelegt, worauf der Operateur den Knoten der Einzelknopfnaht knüpfen kann.

Wenn der Fadenmitnehmer 8 nach dem Vorschieben in seine vorgeschobene Position zurückgezogen wird, so kann dieses Zurückziehen auch weiter als in Fig. 11 dargestellt erfolgen. Der Fadenmitnehmer 8 kann hierbei auch vollständig aus dem Kanal 7 herausgezogen werden und vom Instrument 1 getrennt werden, wobei der Faden 9 vollständig aus dem Fadenkanal 10 des Fadenmitnehmers 8 gezogen wird.

Wenn die herzustellende Einzelknopfnaht mehrere einzelne Fäden mit jeweiligen Knoten umfassen soll, so wird der beschriebene Vorgang wiederholt. Hierbei kann beispielsweise für jeden vorzulegenden Faden 9 eine neue Einrichtung eingesetzt werden, bei der der Fadenmitnehmer 8 mit einem Faden 9 geladen ist. Eine andere Möglichkeit besteht darin, für jeden vorzulegenden Faden 9 den Fadenmitnehmer 8 vollständig aus dem Instrument herauszuziehen und durch einen neuen, mit einem Faden 9 geladenen Fadenmitnehmer zu ersetzen, welcher von proximal in den Kanal 7 eingeschoben wird. Eine weitere Möglichkeit besteht darin, für jeden vorzulegenden Faden 9 den Fadenmitnehmer 8 vollständig aus dem Instrument 1 herauszuziehen und mit einem neuen Faden 9 zu laden.

Das freie Ende des distal der Durchtrittsöffnung 6 liegenden Abschnitts des Maulteils 4, der auch als Zinke bezeichnet werden kann, ist wie beschrieben als Einstechspitze zum Einstechen in das Gewebe 12 ausgebildet. Eine solche Einstechspitze kann hierbei in unterschiedlicher Weise ausgebildet sein. Die Ausbildung kann wie dargestellt in Form einer stumpfen Spitze sein, wobei hier unterschiedliche Abwandlungen möglich sind. Andererseits kann die Ausbildung auch in Form einer schneidenden Spitze sein. Solche schneidenden Spitzen können beispielsweise kegelförmig, pyramidenförmig oder messerschneidenförmig sein.

Wenn eine erfindungsgemäße Einrichtung zum Setzen einer Ligatur verwendet wird, so wird das abzubindende Gewebe in die Maulöffnung 5 des Maulteils 4 eingeführt. Ein Durchstechen des Gewebes kann somit je nach Anwendung entfallen. Eine Ausbildung des distalen, freien Endes des Maulteils 5 in Form einer Einstechspitze ist dann nicht erforderlich. Nachdem das Gewebe in die Maulöffnung 5 eingeführt ist, wird der Fadenmitnehmer 8 von seiner zurückgezogenen in seine vorgeschobene Position verschoben, dann wieder zurückgezogen zumindest bis sein distales Ende im Kanal 7 liegt und in der Folge das Instrument 1 vom abzubindenden Gewebe abgehoben. Nach dem entsprechenden Durchtrennen des Fadens ist der Faden um das abzubindende Gewebe herum gelegt. In der Folge kann das Abbinden erfolgen. Die Schritte erfolgen somit in analoger Weise wie anhand der Fig. 8 bis 12 beschrieben, wobei der Faden aber um das Gewebe herumgezogen wird und nicht durch einen Gewebekanal durchgezogen wird.

Auch eine Kombination einer elastischen Verformbarkeit des die Durchtrittsöffnung 6 zumindest teilweise begrenzenden Materials mit einer elastischen Verformbarkeit der Verdickung 11 ist denkbar und möglich.

Auch eine Auskleidung nur eines Teils des Innenumfangs der Bohrung im Grundteil mit einem elastischen Material ist denkbar und möglich.

Bei dem in den Fig. 1 bis 12 dargestellten Ausführungsbeispiel wird die Einstechspitze des Maulteils 4 quer zur Längsachse des Schaftteils 3 in das Gewebe 12 eingesteckt. Fig. 13 zeigt eine Ausführungsvariante, bei der das Maulteil 4 durch eine Bewegung in Richtung der Längsachse 16 des Schaftteils 3 in das Gewebe 12 einsteckbar ist. Ein an das distale Ende des Maulteils 4 anschließender Endabschnitt des Maulteils 4 schließt hierbei einen Winkel 17 mit der Längsachse 16 ein, der kleiner als 60° ist, vorzugsweise kleiner als 45°.

Beim Gewebe 12, durch das der Faden 9 zur Ausbildung einer Einzelknopfnaht vorgelegt wird oder um das der Faden 9 zum Setzen einer Ligatur geführt wird, kann es sich beispielsweise um einen Muskel, um Parenchym, um eine Sehne oder um eine andere Form von weichem Gewebe (also z.B. nicht Knochen) handeln, das im Körper anzutreffen ist.

Eine erfindungsgemäße Einrichtung kann insbesondere zum Einsatz in der minimalinvasiven Chirurgie, z.B. speziell in der Laparoskopie, ausgebildet sein. Das Maulteil 4 ist hierbei derart ausgebildet, dass es durch einen Trokar durchführbar ist. Trokare weisen üblicherweise Innendurchmesser im Bereich von 5mm bis 25mm auf. Das Schaftteil 3 weist eine ausreichende Länge zum Heranführen bis zur Operationsstelle auf.

Eine Einrichtung gemäß der Erfindung kann insgesamt als Einweginstrument ausgebildet sein. Die Möglichkeit eines vollständigen Herausziehens des Fadenmitnehmers 8 aus dem Kanal 7 kann hier entfallen, da das Instrument 1 mit dem Fadenmitnehmer 8 nach dem Setzen des Fadens 9 entsorgt wird.

Eine zweite Möglichkeit besteht darin, dass das Instrument 1 als Mehrweginstrument ausgebildet ist, der Fadenmitnehmer 8 aber nur einmal verwendbar ist. Das Instrument 1 besteht hierbei aus einem sterilisierbaren, insbesondere dampfsterilisierbaren Material, vorzugsweise Edelstahl. Nach dem Setzen eines Fadens 9 wird der Fadenmitnehmer 8 vom Instrument 1 getrennt und ein neuer Fadenmitnehmer 8, der mit einem Faden 9 geladen ist, wird eingesetzt.

Als weitere Möglichkeit sind sowohl das Instrument 1 als auch der Fadenmitnehmer 8 zusammen mit den an ihm angebrachten Teilen, im gezeigten Ausführungsbeispiel dem Betätigungsteil 13, als Mehrwegteile ausgebildet. Sie sind hierzu sterilisierbar, insbesondere dampfsterilisierbar, und bestehen aus einem zu diesem Zweck geeigneten Material, beispielsweise Edelstahl. Nach dem Setzen eines Fadens 9 wird der Fadenmitnehmer 8 vom Instrument 1 getrennt und mit einem neuen Faden 9 beladen. Für ein jeweiliges Instrument 1 können mehrere Fadenmitnehmer 8 vorhanden sein, die wechselweise eingesetzt werden können.

Unterschiedliche Modifikationen der beschriebenen Ausführungsbeispiele sind denkbar und möglich ohne den Bereich der Erfindung zu verlassen. So könnte beispielsweise das Maulteil 4 auch in anderer Weise als in Form eines gebogenen Endabschnittes eines Schafts ausgebildet sein, beispielsweise als separates, am Schaftteil 3 festgelegtes Teil.

Anstelle der Führung des Fadenmitnehmers 8 durch einen Kanal 7 des Instruments 1 könnte der Fadenmitnehmer 8 auch außen am Schaftteil 3 geführt sein. In der zurückgezogenen Stellung wäre das distale Ende des Fadenmitnehmers 8 hierbei wiederum proximal der Maulöffnung 5 angeordnet, sodass die Maulöffnung 5 freigegeben ist.

Denkbar und möglich wäre auch eine kreisbogenförmige Ausbildung des Schaftteils 3 und des Fadenmitnehmers 8.

### Legende

### zu den Hinweisziffern:

- 1: Instrument
- 2: Griffteil
- 3: Schaftteil
- 4: Maulteil
- 5: Maulöffnung
- 6: Durchtrittsöffnung
- 7: Kanal
- 8: Fadenmitnehmer
- 9: Faden
- 10: Fadenkanal
- 11: Verdickung
- 12: Gewebe
- 13: Betätigungsteil
- 16: Längsachse
- 17: Winkel

## Patentansprüche

1. Chirurgische Einrichtung umfassend ein Instrument (1), das ein Schaftteil (3) und ein an einem distalen Ende des Schaftteils (3) angeordnetes Maulteil (4) mit einer Maulöffnung (5) aufweist, einen Fadenmitnehmer (8), der vom Schaftteil (3) geführt ist und von einer zurückgezogenen Position, in der er die Maulöffnung (5) freigibt, in eine vorgeschobene Position, in der er die Maulöffnung (5) überquert und mit einem distalen Ende durch eine distal der Maulöffnung (5) gelegene Durchtrittsöffnung (6) des Maulteils (4) ragt, verschiebbar ist, und einen Faden (9), der mittels des Fadenmitnehmers (8) durch ein menschliches oder tierisches Gewebe (12), in dem eine Naht anzubringen ist, oder um ein menschliches oder tierisches Gewebe (12) herum, um das eine Ligatur anzulegen ist, führbar ist, wobei der am Faden (9) endseitig mit einer Verdickung (11) versehen ist, die vom Fadenmitnehmer (8) beim Verschieben des Fadenmitnehmers (8) von seiner zurückgezogenen in seine vorgeschobene Position mitgenommen wird, **dadurch gekennzeichnet, dass** die am Faden (9) angeordnete endseitige Verdickung (11) vom Material des Fadens (9) selbst gebildet wird, wobei die Verdickung (11) beim Verschieben des Fadenmitnehmers (8) von seiner zurückgezogenen in seine vorgeschobene Position unter zumindest teilweise elastischer Verformung der Verdickung (11) durch die Durchtrittsöffnung (6) des Maulteils (4) gedrückt wird.

2. Chirurgische Einrichtung nach Anspruch 1, **dadurch gekennzeichnet, dass** der Fadenmitnehmer (8) in einem durch das Schaftteil (3) verlaufenden inneren Kanal (7) geführt ist und in seiner vorgeschobenen Position mit einem Endabschnitt, der die Maulöffnung (5) überquert, aus dem inneren Kanal (7) herausragt.

3. Chirurgische Einrichtung nach Anspruch 2, **dadurch gekennzeichnet, dass** der Fadenmitnehmer (8) vollständig aus einem proximalen Ende des inneren Kanals (7) des Schaftteils (3) herausziehbar ist.

4. Chirurgische Einrichtung nach einem der Ansprüche 1 bis 3, **dadurch gekennzeichnet, dass** der Fadenmitnehmer (8) röhrchenförmig ausgebildet ist.

5. Chirurgische Einrichtung nach einem der Ansprüche 1 bis 4, **dadurch gekennzeichnet, dass** der Faden (9) beim Verschieben des Fadenmitnehmers (8) von der zurückgezogenen Position in die vorgeschobene Position durch einen den Fadenmitnehmer (8) durchsetzenden inneren Fadenkanal (10) verläuft, der an einem distalen Ende des Fadenmitnehmers (8) mündet, vor dem die Verdickung (11) des Fadens liegt.

6. Chirurgisches Instrument nach einem der Ansprüche 1 bis 5, **dadurch gekennzeichnet, dass** das Maulteil (4) von einem gebogenen Endabschnitt eines Schaftes des Instruments (1) gebildet wird, der auch das Schaftteil (3) des Instruments (1) bildet.

7. Chirurgische Einrichtung nach einem der Ansprüche 1 bis 6, **dadurch gekennzeichnet, dass** sich die Maulöffnung (5) des Maulteils (4) bezogen auf die Längserstreckung des Schaftteils (3) zur Seite hin öffnet.

8. Chirurgische Einrichtung nach einem der Ansprüche 1 bis 7, **dadurch gekennzeichnet, dass** ein distal der Maulöffnung (5) liegendes Ende des Maulteils (4) als Einsteckspitze zum Einstechen in das Gewebe (12) ausgebildet ist, in dem die Naht anzubringen ist.

9. Chirurgische Einrichtung nach einem der Ansprüche 1 bis 8, **dadurch gekennzeichnet, dass** sich die Durchtrittsöffnung (6) im Maulteil (4) zur Maulöffnung (5) hin erweitert.

10. Chirurgische Einrichtung nach einem der Ansprüche 1 bis 9, **dadurch gekennzeichnet, dass** sich beim Durchdrücken der Verdickung (11) durch die Durchtrittsöffnung (6) des Maulteils (4) beim Verschieben des Fadenmitnehmers (8) von seiner zurückgezogenen in seine vorgeschobene Position auch ein die Durchtrittsöffnung (6) im Maulteil (4) zumindest teilweise begrenzendes Material zumindest teilweise elastisch verformt.

## Claims

1. Surgical device comprising an instrument (1), the instrument (1) having a shank portion (3) and a mouth portion (4), which latter is arranged at a distal end of the shank portion (3) and has a mouth aperture (5), having a filament entrainer (8) which is guided by the shank portion (3) and which is displaceable from a withdrawn position in which it leaves the mouth aperture (5) open, to an advanced position in which it bridges the mouth aperture (5) and in which a distal end thereof projects through a through-opening (6) in the mouth portion (4) which is situated distally of the mouth aperture (5), and having a filament (9) which can be guided by means of the filament entrainer (8) through human or animal tissue (12) in which a suture is to be made, or around human or animal tissue (12) which is to be ligated, the filament (9) being provided at the end with an enlargement (11) which is entrained by the filament entrainer (8) when said filament entrainer (8) is displaced from its withdrawn to its advanced position, **characterised in that** the enlargement (11) arranged at the end of the filament (9) is formed by the material of the filament (9) itself, the enlargement (11) being pressed through the through-opening (6) in the mouth portion (4) when the filament entrainer (8) is displaced from its withdrawn to its advanced position, accompanied by elastic deformation of at least part of said enlargement (11).

2. Surgical device according to claim 1, **characterised in that** the filament entrainer (8) is guided in an inner passage (7) extending through the shank portion (3) and, when it is in its advanced position, an end portion thereof, which bridges the mouth aperture (5), projects from the inner passage (7).

3. Surgical device according to claim 2, **characterised in that** the filament entrainer (8) can be completely withdrawn from a proximal end of the inner passage (7) in the shank portion (3).

4. Surgical device according to one of claims 1 to 3, **characterised in that** the filament entrainer (8) is in the form of a slim tube.

5. Surgical device according to one of claims 1 to 4, **characterised in that**, when the filament entrainer (8) is displaced from the withdrawn position to the advanced position, the filament (9) extends through an inner filament passage (10) passing through the filament entrainer (8) which opens at a distal end of the filament entrainer (8) ahead of which the enlargement (11) in the filament is situated.

6. Surgical instrument according to one of claims 1 to 5, **characterised in that** the mouth portion (4) is formed by a bent end portion of a shank of the instrument (1), which shank also forms the shank portion (3) of the instrument (1).

7. Surgical device according to one of claims 1 to 6, **characterised in that** the mouth aperture (5) of the mouth portion (4) opens sideways from the longitudinal extent of the shank portion (3).

8. Surgical device according to one of claims 1 to 7, **characterised in that** an end of the mouth portion (4) which is situated distally of the mouth aperture (5) takes the form of a puncturing point for insertion into the tissue (12) in which the suture is to be made.

9. Surgical device according to one of claims 1 to 8, **characterised in that** the through-opening (6) in the mouth portion (4) widens towards the mouth aperture (5).

10. Surgical device according to one of claims 1 to 9, **characterised in that**, as the enlargement (11) is pressed through the through-opening (6) in the mouth portion (4) when the filament entrainer (8) is displaced from its withdrawn to its advanced position, a material which forms at least part of the boundary of the through-opening (6) in the mouth portion (4) is also at least partially deformed elastically.

## Revendications

1. Dispositif chirurgical comprenant un instrument (1) qui comporte une partie de tige (3) et une partie formant mâchoire (4) située à l'extrémité distale de la partie de tige (3) et ayant une ouverture de mâchoire (5), un entraineur de fil (8) qui est guidé par la partie de tige (3) et est mobile en translation entre une position rétractée dans laquelle il libère l'ouverture de mâchoire (5) et une position déployée dans laquelle il traverse l'ouverture de mâchoire (5) et pénètre, par une extrémité distale au travers d'une ouverture de passage distale (6) de l'ouverture de mâchoire (5), et un fil (9) qui peut être guidé au moyen de l'entraineur de fil (8) au travers d'un tissu humain ou animal (12) dans lequel une suture doit être effectuée, ou, autour d'un tissu humain ou animal (12) autour duquel une ligature doit être effectuée, à l'extrémité du fil (9) étant prévue une surépaisseur (11) qui est prise par l'entraineur de fil (8) lors de sa translation entre sa position rétractée et sa position déployée,
**caractérisé en ce que**
la surépaisseur (11) située à l'extrémité du fil (9) est formée par le matériau du fil (9) lui-même, lors de la translation de l'entraineur de fil (8) de sa position rétractée à sa position déployée, la surépaisseur (11) étant poussée au travers de l'ouverture de passage (6) de la partie formant mâchoire (15) en étant au moins partiellement déformée élastiquement.

2. Dispositif chirurgical conforme à la revendication 1,
**caractérisé en ce que**
l'entraineur de fil (8) est guidé dans un canal interne (7) s'étendant au travers de la partie de tige (3), et dépasse du canal interne (7) par un segment d'extrémité qui traverse l'ouverture de mâchoire (5) dans sa position préalablement glissée.

3. Dispositif chirurgical conforme à la revendication 2,
**caractérisé en ce que**
l'entraineur de fil (8) peut être totalement extrait de l'extrémité proximale du canal interne (7) de la partie de tige (3).

4. Dispositif chirurgical conforme à l'une des revendications 1 à 3,
**caractérisé en ce que**
l'entraineur de fil (8) est réalisé sous la forme d'un petit tube.

5. Dispositif chirurgical conforme à l'une des revendications 1 à 4,
**caractérisé en ce que**
lors de la translation de l'entraineur de fil (8) de sa position rétractée à sa position déployée, le fil (9) s'étend au travers d'un canal de fil interne (10) traversant l'entraineur de fil (8) qui débouche à l'extrémité distale de cet entraineur de fil (8) à l'avant de laquelle est située la surépaisseur (11) du fil.

6. Dispositif chirurgical conforme à l'une des revendications 1 à 5,
**caractérisé en ce que**
la partie formant mâchoire (4) est formée par un segment d'extrémité courbe d'une tige de l'instrument (1) qui forme également la partie de tige (3) de l'instrument (1).

7. Dispositif chirurgical conforme à l'une des revendications 1 à 6,
**caractérisé en ce que**
l'ouverture de mâchoire (5) de la partie formant mâchoire (5) s'ouvre latéralement par rapport à l'extension longitudinale de la partie de tige.

8. Dispositif chirurgical conforme à l'une des revendications 1 à 7 ;
**caractérisé en ce que**
l'extrémité distale de la partie formant mâchoire (4) par rapport à l'ouverture de mâchoire (5) est réalisée sous la forme d'une pointe d'insertion destinée à être piquée dans le tissu (12) dans lequel la suture doit être effectuée.

9. Dispositif chirurgical conforme à l'une des revendications 1 à 8,
**caractérisé en ce que**
l'ouverture de passage (6) de la partie formant mâchoire (4) s'élargit vers l'ouverture de mâchoire (5).

10. Dispositif chirurgical conforme à l'une des revendications 1 à 9,
**caractérisé en ce que**
lors du passage à force de la surépaisseur (11) au travers de l'ouverture de passage (6) de la partie formant mâchoire (4) lors de la translation de l'entraineur de fil (8) de sa position rétractée à sa position déployée, le matériau limitant au moins partiellement l'ouverture de passage (6) dans la partie formant mâchoire (4) se déforme également au moins partiellement élastiquement.
